Europäisches Patentamt

European Patent Office

Office européen des brevets

(11) Numéro de publication : **0 517 608 A1**

# (12) DEMANDE DE BREVET EUROPEEN

(21) Numéro de dépôt : 92401563.9

(51) Int. Cl.⁵ : **A61B 17/34**

(22) Date de dépôt : **05.06.92**

(30) Priorité : **07.06.91 FR 9106978**

(43) Date de publication de la demande :
**09.12.92 Bulletin 92/50**

(84) Etats contractants désignés :
**AT BE CH DE DK ES FR GB GR IT LI LU MC NL PT SE**

(71) Demandeur : **Gilbert, Alain**
**14 rue Broca**
**F-75005 Paris (FR)**

(71) Demandeur : **Merle, Michel**
**5 Allée des Roches**
**F-54000 Nancy (FR)**

(72) Inventeur : **Gilbert, Alain**
**14 rue Broca**
**F-75005 Paris (FR)**
Inventeur : **Merle, Michel**
**5 Allée des Roches**
**F-54000 Nancy (FR)**

(74) Mandataire : **Martin, Jean-Jacques**
**Cabinet REGIMBEAU 26, Avenue Kléber**
**F-75116 Paris (FR)**

(54) **Guide-introducteur pour opération orthopédique.**

(57) la présente invention concerne un guide-introducteur pour opération de chirurgie orthopédique, notamment une opération de soulagement du canal carpien, caractérisé par le fait qu'il est formé d'une pièce rigide (100) comprenant deux parties : une palette allongée (110), conque pour être engagée par une incision, dans un canal, et un manche (150) raccordé à l'extrémité proximale (120) de la palette, ce manche (150) étant incliné par rapport à la palette (110) afin de faciliter un pivotement de la palette (110) autour de son axe longitudinal (112), après insertion dans le canal, pour ménager ainsi un passage libre facilitant l'insertion d'une canule dans le canal.

FIG.1

La présente invention concerne un dispositif pour opérations de chirurgie orthopédique, en particulier pour une opération de soulagement du canal carpien.

On a proposé depuis de nombreuses années diverses techniques de soulagement du canal carpien par section du ligament annulaire du carpe (ci-après ligament carpien), lorsque celui-ci comprime de façon néfaste le nerf sous-jacent.

Les premières méthodes proposées à cet effet, depuis plus de 50 ans, consistaient à ouvrir largement le poignet du patient pour accéder ainsi au ligament carpien et sectionner celui-ci lors d'une opération ouverte.

On a cependant proposé depuis plusieurs années une méthode moins traumatisante, sous endoscopie, qui consiste essentiellement à pratiquer une petite incision à la base du poignet, à insérer une canule fendue sur toute sa longueur, dans le canal carpien, par cette incision, jusqu'à faire émerger l'extrémité distale de la canule dans la paume de la main, puis à engager des outils de coupe appropriés dans la canule, par une extrémité de celle-ci, pour sectionner le ligament carpien, en contrôlant ces opérations à l'aide d'un endoscope engagé dans la canule par la seconde extrémité de celle-ci.

Cette technique est décrite par exemple dans la revue "The Journal of Arthroscopic and Related Surgery", vol. 5, n° 1, 1989, pages 19 à 24, "Endocospic Release of the Carpal ligament : A New Technique for Carpal Tunnel Syndrome", James C. Y. Chow, M.D.

Toutefois, la technique d'intervention sur le ligament carpien, sous endoscopie, ainsi proposée n'a pas jusqu'ici donné pleinement satisfaction.

Les chirurgiens tentant de la mettre en oeuvre constatent en effet fréquemment qu'il est difficile d'engager la canule dans le canal carpien sans traumatiser les tissus, nerfs, tendons et ligaments environnants. Par ailleurs, la difficulté de protéger tendons et nerfs a entraîné des complications.

Pour cette raison, la technique d'opération du ligament carpien sous endoscopie, bien qu'elle soit apparue très prometteuse dans un premier temps, tend à être abandonnée de nos jours.

La présente invention a pour but d'améliorer la situation en proposant de nouveaux moyens qui permettent d'éliminer les inconvénients de la technique connue.

Ce but est atteint selon la présente invention grâce à un guide-introducteur formé d'une pièce rigide comprenant deux parties : une palette allongée, conçue pour être engagée par une incision, dans le canal carpien, et un manche raccordé à l'extrémité proximale de la palette, ce manche étant incliné par rapport à la palette afin de faciliter un pivotement de la palette autour de son axe longitudinal, après insertion dans le canal carpien, pour éloigner le ligament carpien du fond osseux et écarter les nerfs et tendons environnants, et ménager ainsi un passage libre facilitant l'insertion d'une canule dans le canal carpien.

Selon une autre caractéristique avantageuse de la présente invention, la palette possède une creusure longitudinale apte à servir de guide à la canule.

Selon une autre caractéristique avantageuse de la présente invention, la creusure longitudinale est formée d'une concavité généralement cylindrique.

D'autres caractéristiques, buts et avantages de la présente invention apparaîtront à la lecture de la description détaillée qui va suivre et en regard des dessins annexés donnés à titre d'exemple non limitatif et sur lesquels :

- la figure 1 représente une vue schématique en perspective d'un guide introducteur conforme à la présente invention,
- la figure 2 représente une vue latérale du même guide introducteur,
- la figure 3 représente schématiquement le guide introducteur en place dans le canal carpien, et une canule fendue engagée dans celui-ci,
- les figures 4 à 11 illustrent schématiquement les diverses étapes d'une opération de soulagement du canal carpien mettant en oeuvre le guide introducteur conforme à la présente invention.

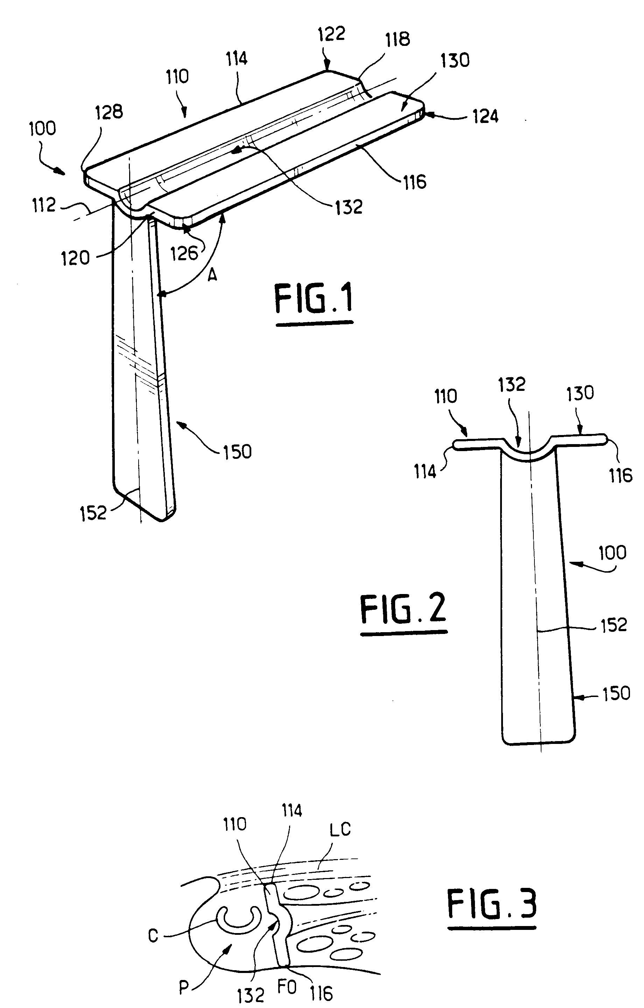

On aperçoit sur les figures 1 et 2, un guide introducteur 100 conforme à la présente invention comprenant deux parties principales : une palette 110 et un manche 150.

Le guide introducteur 100 est réalisé en matériau rigide, de préférence en métal. Il peut s'agir de tout métal classique approprié, le cas échéant revêtu d'un matériau noble. Il peut également être réalisé en tout autre matériau dur, par exemple en plastique.

De préférence, le guide introducteur 100 est formé par matriçage et pliage à partir d'un flan plan.

La palette 110 est allongée et de contour sensiblement rectangulaire.

Son axe longitudinal est référencé 112 sur la figure 1. Les bords longitudinaux de la palette 110 sont référencés 114 et 116. Ces bords longitudinaux 114, 116 sont parallèles entre eux et parallèles à l'axe longitudinal 112.

Le bord distal de la palette 110 est référencé 118. Son bord proximal est référencé 120. Le bord distal 118 et le bord proximal 120 sont sensiblement parallèles entre eux et orthogonaux aux bords longitudinaux 114, 116.

Les bords longitudinaux 114, 116, le bord distal 118 et le bord proximal 120 sont généralement arrondis, pour ne pas être coupants. Toutefois, de préférence, les bords longitudinaux 114, 116 sont rugueux afin de faciliter le décollement des tissus lors du pivotement de la palette, comme cela sera précisé par la suite.

On notera à l'examen de la figure 1, que de préférence les bords longitudinaux 114, 116 sont raccordés au bord distal 118 et au bord proximal 120 par des angles arrondis 122, 124, 126, 128.

Comme indiqué précédemment, selon une caractéristique importante de la présente invention, la palette 110 est munie sur l'une 130 de ses faces principales d'une creusure longitudinale 132. Celle-ci sert de guide à la canule fendue lors de l'introduction de cette dernière dans le passage libre formé dans le canal carpien.

La creusure 132 est placée à mi-largeur de la palette, c'est-à-dire à mi-distance des bords longitudinaux 114, 116. Elle est formée d'une déformation de la palette constante sur toute la longueur de celle-ci. Cette déformation a la forme générale d'une concavité cylindrique centrée sur l'axe longitudinal 112 ou un axe parallèle à cet axe longitudinal 112.

De préférence, la creusure 132 est sensiblement complémentaire d'une partie de la surface externe de la canule fendue utilisée.

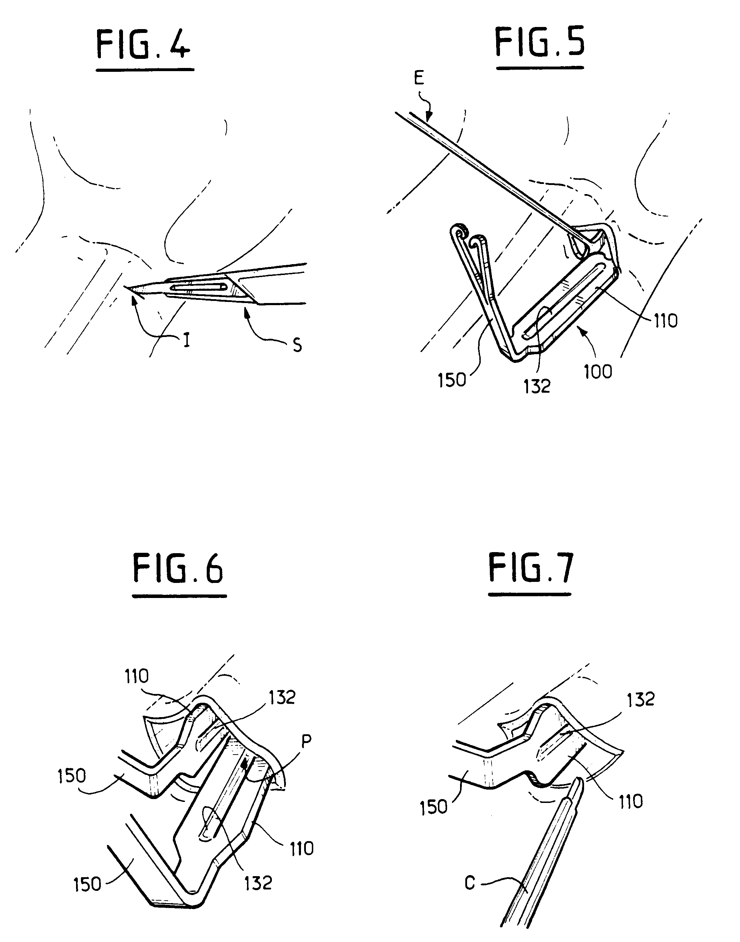

Le manche 150 peut faire l'objet de nombreux modes de réalisation. Selon la figure 1, il est formé d'un élément monobloc généralement rectangulaire. Selon la variante de réalisation représentée sur les figures 5, 8, 9 et 10, le manche 150 a la forme générale d'une fourche à deux branches. Bien entendu, l'invention n'est pas limitée à ces deux modes de réalisation.

Le manche 150 se raccorde au bord proximal 120 de la palette.

Le manche 150 est de préférence plan. Il est de plus incliné par rapport à la palette. L'angle d'inclinaison entre le manche 150 et la palette 110 peut faire l'objet de nombreuses variantes. De façon avantageuse, l'angle d'inclinaison A entre le manche 150 et la palette 110 est de l'ordre de 90°. Sur la figure 1, on a référencé 152 l'axe longitudinal du manche 150. Cet axe 152 est coplanaire et sécant de l'axe 112.

Le manche 150 est placé sur la seconde face principale 132 de la palette 130, soit côté convexe de la creusure 132.

Le guide introducteur 100 conforme à l'invention peut faire l'objet de nombreuses variantes, quant à ses dimensions.

A titre d'exemple non limitatif, la longueur de la palette 110, considérée selon l'axe 112 est de l'ordre de 6 à 7 cm. Il est de préférence prévu un jeu de plusieurs guides introducteur 100 possédant des palettes 110 de largeur variable comprise typiquement entre 7 et 15 mm, afin d'adapter l'outil utilisé à la taille de la main du patient opéré. La largeur de la creusure est typiquement de 4 à 7 mm.

On va maintenant décrire le processus d'intervention mettant en oeuvre le guide introducteur 100.

La main du patient est posée à plat, paume dirigée vers le haut et les doigts en extension.

Comme représenté sur la figure 4, la première opération consiste, à pratiquer une incision I à l'aide d'un scalpel 5 à la base du poignet.

Une fois cette incision pratiquée, un guide introducteur 100 conforme à la présente invention est introduit à plat dans le canal carpien, c'est-à-dire palette 110 sensiblement parallèle à la face interne du poignet. Cette opération introduction du guide introducteur 100 peut être facilité le cas échéant par un écarteur E comme représenté sur la figure 5.

Le guide introducteur 100 est alors pivoté d'environ 90° autour de l'axe longitudinal 112 de la palette 110, comme représenté sur la figure 6, par actions sur le manche 150 pour séparer le ligament carpien du fond osseux, écarter les nerfs et tendons environnants et former un passage P dans le canal carpien. La formation de ce passage peut être facilitée en engagement un second guide introducteur 100 comme représenté sur la figure 6, pour décoller plus aisément les tissus.

La palette 110 est alors sensiblement perpendiculaire à la face interne du poignet comme représenté sur la figure 6.

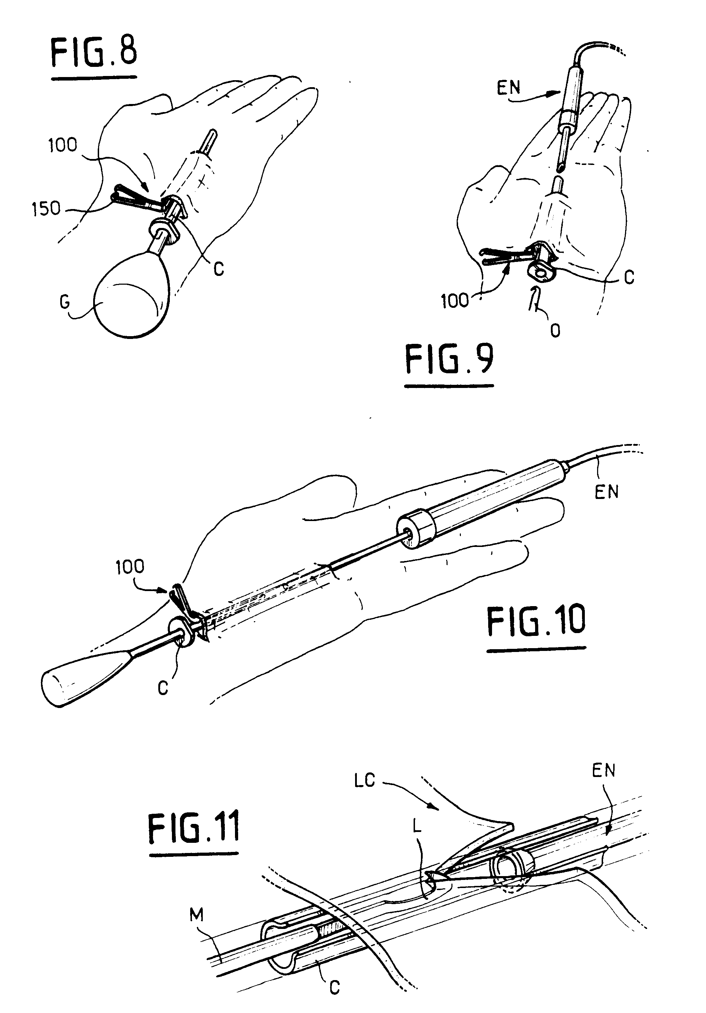

Une canule C fendue sur toute sa longueur peut alors être introduite dans le passage P comme représenté sur la figure 7. De préférence, lors de cette phase d'introduction, un guide rigide G est placé dans la canule C. Le guide G possède une nervure longitudinale introduite dans la fente de la canule C, de sorte qu'une rotation du guide G autour de son axe permette d'orienter la canule C et placer sa fente en regard du ligament carpien. Cette étape est illustrée sur la figure 8.

Au cours de cette phase d'introduction de la canule C, le chirurgien peut glisser la canule C dans la creusure 132 de la palette 110, ce qui facilite le guidage de la canule C et évite en toute sécurité de traumatiser les milieux environnants.

Le guide G peut alors être retiré. Comme illustré sur la figure 9, le chirurgien peut introduire des outils de coupe O par une première extrémité de la canule et un endoscope EN par la seconde extrémité de celui-ci.

Selon la figure 9, les outils O sont introduits par l'extrémité proximale de la canule C tandis que l'endoscope EN est introduit par l'extrémité distale de la canule C. La disposition inverse peut être retenue. Si nécessaire, au cours d'une même opération, le chirurgien peut également inverser la disposition des outils de coupe O et de l'endoscope EN.

Les opérations de coupe du ligament carpien LC sont illustrées sur les figures 10 et 11. Ces opérations de coupe connues en elles-mêmes du chirurgien spécialisé ne seront pas décrites plus en détail par la suite. On notera simplement que le plus souvent elles nécessitent l'utilisation successive de différents instruments.

On aperçoit en particulier sur la figure 11, l'extrémité de l'endoscope EN permettant d'observer la coupe du ligament carpien LC à l'aide d'une lame incurvée L placée d'un manche M allongé.

Une fois la coupe du ligament achevée, les outils de coupe, l'endoscope EN, la canule C et le guide introducteur 100 sont retirés.

On a représenté schématiquement sur la figure 3, un guide introducteur 100 conforme à la présente invention dans le canal carpien, entre le fond osseux FO et le ligament carpien LC. Sur la même figure 3, on a référencé P le passage libre ménagé par le guide 100 et la canule C.

Bien entendu la présente invention n'est pas limitée au mode de réalisation particulier qui vient d'être décrit mais s'étend à toutes variantes conformes à son esprit.

Selon la description qui précède, la creusure 132 est située à mi-distance des bords longitudinaux 114, 116. On peut cependant prévoir une creusure 132 décalée vers un bord longitudinal de la palette. Ce décalage peut tenir compte de la focale de l'optique de l'endoscope EN.

De plus selon la description qui précède la creusure 132 s'étend sur toute la longueur de la palette. On peut cependant prévoir que la creusure ne s'étende que sur une partie limitée de la palette 110, adjacente au manche 150, par exemple sur 1cm.

## Revendications

1. Guide-introducteur pour opération de chirurgie orthopédique, notamment une opération de soulagement du canal carpien, caractérisé par le fait qu'il est formé d'une pièce rigide (100) comprenant deux parties : une palette allongée (110), conçue pour être engagée par une incision, dans un canal, et un manche (150) raccordé à l'extrémité proximale (120) de la palette, ce manche (150) étant incliné par rapport à la palette (110) afin de faciliter un pivotement de la palette (110) autour de son axe longitudinal (112), après insertion dans le canal, pour ménager ainsi un passage libre facilitant l'insertion d'une canule dans le canal.

2. Guide-introducteur selon la revendication 1, caractérisé par le fait que la palette (110) possède une creusure (132) longitudinale apte à servir de guide à la canule.

3. Guide-introducteur selon l'une des revendications 1 ou 2, caractérisé par le fait que la creusure (132) longitudinale est formée d'une concavité généralement cylindrique.

4. Guide-introducteur selon l'une des revendications 2 ou 3, caractérisé par le fait que la creusure (132) s'étend sur toute la longueur de la palette (110).

5. Guide-introducteur selon l'une des revendications 2 ou 3, caractérisé par le fait que la creusure (132) s'étend sur une partie de la longueur de la palette (110) adjacente au manche (150).

6. Guide-introducteur selon l'une des revendications 2 à 5, caractérisé par le fait que la creusure (132) est formée sensiblement à mi-largeur de la palette (110).

7. Guide-introducteur selon l'une des revendications 2 à 5, caractérisé par le fait que la creusure (132) est décalée vers un bord longitudinal (114, 116) de la palette (110).

8. Guide-introducteur selon l'une des revendications 2 à 7, caractérisé par le fait que la creusure (132) possède une section constante sur toute la longueur de la palette (110).

9. Guide-introducteur selon l'une des revendications 2 à 8, caractérisé par le fait que la creusure (132) est sensiblement complémentaire de l'enveloppe externe de la canule.

10. Guide-introducteur selon l'une des revendications 2 à 9, caractérisé par le fait que le manche (150) est incliné d'environ 90° par rapport à la palette (110).

11. Guide-introducteur selon l'une des revendications 2 à 10, caractérisé par le fait que le manche (150) est raccordé sur la palette (110) côté convexe de la creusure (132).

12. Guide-introducteur selon l'une des revendications 1 à 11, caractérisé par le fait que la longueur de la palette (110) est comprise entre 6 et 7 cm.

13. Guide-introducteur selon l'une des revendications 1 à 12, caractérisé par le fait que la largeur de la palette (110) est comprise entre 7 à 15 mm.

FIG.1

FIG.2

FIG.3

FIG.4

FIG.5

FIG.6

FIG.7

## FIG.8

## FIG.9

## FIG.10

## FIG.11

EP 0 517 608 A1

Office européen
des brevets

**RAPPORT DE RECHERCHE EUROPEENNE**

Numero de la demande

EP 92 40 1563

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | US-A-4 883 059 (STEDMAN) * colonne 1, ligne 36 - ligne 37; figures 2,3 * --- | 1 | A61B17/34 |
| A | EP-A-0 101 781 (HERAEUS) * figure 1 * --- | 1 | |
| A | WO-A-9 014 041 (COUGHENOUR) * page 6, ligne 7; figure 3 * --- | 1 | |
| A | US-A-4 827 910 (MATTHEWS, III) * abrégé; figures 1,2 * ----- | 1 | |

DOMAINES TECHNIQUES
RECHERCHES (Int. Cl.5 )

A61B

Le présent rapport a été établi pour toutes les revendications

| Lieu de la recherche | Date d'achèvement de la recherche | Examinateur |
|---|---|---|
| LA HAYE | 20 AOUT 1992 | BARTON S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.......................................................................................
& : membre de la même famille, document correspondant

EPO FORM 1503 03.82 (P0402)

8